# EUROPEAN PATENT APPLICATION

(11) **EP 0 949 508 A1**
(43) Date of publication of application: **13.10.1999**
(21) Application number: 99610026.9
(22) Date of filing: 07.04.1999
(51) Int. Cl.: G01N 33/569, C07K 14/20, A61K 39/02

(54) **Method, antigen complex and kit for diagnosing Lyme borreliosis**

(30) Priority: 08.04.1998 DK 51698
(71) Applicant: DAKO A/S, 2600 Glostrup (DK)
(72) Inventor: Staffeldt Schou, Ole, Svogerslev, 4000 Roskilde (DK); Winther, Lars, 2300 Kobenhavn S (DK); Stender, Henrik, Waltham, MA 02451 (US)
(74) Representative: Halberg, Kristian

(57) **Abstract**

A diagnostic method is disclosed which utilises an antigen complex comprising a carrier to which is attached a plurality of *Borrelia* OspC peptides and optionally other *Borrelia* peptides. The antigen complex is used for determination of IgM antibodies against *B*. *burgdorferi sensu lato* . The antigen complex comprises a sufficient number of peptides having affinity for a relevant epitope on the IgM antibody to be detected to allow the antigen complex to bind to the IgM antibody without being removed during a washing step. The *Borrelia* flagellum is another important immunodominant antigen. The simultaneous determination of antibodies against *Borrelia* OspC and flagellum is also disclosed. The method is particularly useful for diagnosing Lyme borreliosis in the early stages.

## Description

The present invention relates to a method for the determination of IgM antibodies against *Borrelia burgdorferi* (*B. burgdorferi*) *sensu lato* in a sample of human or animal fluid. The invention further relates to an antigen complex for use in said method and a kit comprising the antigen complex. The present invention is particularly useful for diagnosing Lyme borreliosis in the early stages.

### BACKGROUND OF THE INVENTION

Lyme borreliosis was described in the late 1970's as a unique grouping of arthritic symptoms in patients from Lyme, Conneticut. The disease was shown to be caused by infection with *B. burgdorferi,* a spirochete, following transmission by deer ticks. Since 1985 the disease complex due to *B. burgdorferi* has been called Lyme borreliosis. Lyme borreliosis is now recognised as the most common vector-borne human disease in Europe and North America. Impairment of the nervous system, named Lyme neuroborreliosis, is, at least in Europe, the predominant cause of significant morbidity due to *B. burgdorferi.*

Recognising Lyme borreliosis in the early stages can be difficult, because patients may not manifest the characteristic rash (*erythema migrans*) and may have only non-specific flu-like symptoms. Diagnosis of Lyme borreliosis in its early stages is clinically important because timely institution of appropriate antibiotic treatment can prevent the serious complications from this potentially chronic infection.

Direct detection of *B. burgdorferi* in body fluids and tissues is possible but difficult, and these methods are not sensitive. *B. burgdorferi* may be cultured from clinical samples, but it is time consuming and the sensitivity is low. It is possible to use PCR (Polymerase Chain Reaction), but again with unsatisfactory sensitivity. Thus, the laboratory diagnosis of Lyme borreliosis still depends mainly on detection of *B. burgdorferi* antibodies in blood and CSF.

Even though laboratory diagnosis of Lyme borreliosis has been possible since the discovery in 1982 of the etiological agent, *B. burgdorferi,* the diagnostic performance of Lyme borreliosis serology is still not satisfactory. Serological tests were for a long time based on crude cell antigen preparations as test antigen. However, due to cross reactions caused by other microorganisms, the specificity was very low.

In an attempt to improve the diagnostic performance, Western blotting has been applied. This method determines the immunoreactivity to the individual proteins of B. *burgdorferi,* which are electrophoretically separated according to their molecular mass. Western blotting is very suitable for identification of immunodominant B. burgdorferi antigens, and according to Western blotting studies there are mainly two B. burgdorferi antigens that meet the essential criteria of eliciting an early and strong antibody reponse in the majority of Lyme borreliosis patients. These are the B. burgdorferi flagellum protein subunit, flagellin (p41) and the outer surface protein C, OspC (p23). Flagellin is also an immunodominant antigen in late stages of Lyme borreliosis. However, Western blotting is laboreous to standardise and not suited as a routine laboratory test.

Various flagellum-based Enzyme-Linked Immunosorbent Assays (ELISAs) have been established and are now commonly used for routine analysis for Borrelia infections (e.g. Hansen et al., ref. 1 and ref. 2).

In WO 97/42221 (ref. 3) it is suggested to increase the sensitivity of diagnosis of the early stages of Lyme borreliosis by combining the results from the currently available immunoassays based on purified *Borrelia* flagella with results from a method detecting antibodies against the *Borrelia* OspC protein and an increase of 15 % was reported. The immunological sensitivity of various synthetic peptides derived from OspC were compared to the sensitivity obtained with full length recombinant OspC. The serodiagnostic sensitivity using synthetic peptides comprising the C-terminal end compared to full-length OspC was reported to be comparable.

The use of synthetic peptides helps simplify the preparation and purification steps of the components of an assay and thus help standardise the assay. Furthermore, the use of synthetic peptides in an immunoassay may lead to a decrease in the cross-reactivity with antibodies raised against other antigens as the synthetic peptides have a much reduced number of epitopes which may cause cross-reactivity.

WO 97/42221 relates to various immunoassays using OspC peptides but only indirect ELISA is exemplified. In indirect ELISA, the test antigen adsorbed to the solid support binds antigen-specific antibodies of all isotypes in an amount positively related to their concentration and avidity in the test sample. The bound specific antibody is subsequently detected with a second antibody which may be class specific. Due to the binding of all classes and isotypes of sample antibodies to the test antigen adsorbed on the solid support, the different classes and isotypes will compete with each other for antigenic sites. This is a particular drawback when immunoglobulin classes or isotypes present in low amounts are to be detected. IgM antibodies are often present in a low amount. Furthermore, indirect ELISA for IgM may give false-positive results due to interference from rheumatoid factor (RF) of the IgM class. In the presence of antigen-specific IgG, the RF may bind to the solid support through IgG and give rise to a false-positive result.

The disadvantages associated with the indirect IgM ELISAs can be overcome by using a µ-capture principle whereby patient IgM antibodies are captured by anti-IgM immobilised on a solid support. Following the capturing of the patient IgM antibodies to the solid support, non-bound components of the sample such as IgG are removed by a washing step.

A three-step µ-capture ELISA for detection of IgM antibodies to the native flagellum of *B. burgdorferi* has been described in ref. 2. In this assay, a complex of biotinylated purified *B. burgdorferi* flagella and avidin peroxidase is used as test antigen. An increased signal-to-noise ratio was reported whereby a significant better quantitative discrimination of individual positive measurements from the cut-off was obtained. So far no µ-capture assay using *Borrelia* OspC peptides have been reported.

Even though the performance of Lyme serology for diagnosis of early stages of the disease has increased recently, at latest by performing parallel tests with the native flagellum as well as with OspC as antigen, further improvements are needed to increase the sensitivity, avoid interference from RF and provide a labour and cost saving method that is suited for routine testing.

### SUMMARY OF THE INVENTION

The present inventors have now succeeded in developing a µ-capture assay based on OspC peptides. Besides, the inventors have shown that an embodiment of a µ-capture assay according to the invention, wherein an antigen complex comprising *Borrelia* OspC peptides and a labelled native *Borrelia* flagellum acting as carrier for the peptides are used, provides a means for the simultaneous detection of antibodies against *Borrelia* OspC and the flagellum.

In the broadest aspect, the invention relates to a method for the determination of IgM antibodies against *B. burgdorferi sensu lato* in a sample of human or animal fluid which method comprises the steps of
(i) contacting any sample antibodies to be detected with anti-IgM immobilised to a solid support,
(ii) optionally separating the solid support from the liquid phase,
(iii) contacting the antibodies bound to the solid support with an antigen complex comprising a carrier to which is attached one or more sets of *Borrelia* OspC peptides and optionally one or more sets of other *Borrelia* peptides, each set comprising a plurality of *Borrelia* peptides representing the same antibody binding epitope and binding to IgM molecules, which antigen complex is or will be labelled, and
(iv) determining the presence, if any, of antibodies against *B. burgdorferi sensu lato.*

In a further aspect, the present invention relates to a method for the determination of IgM antibodies against *B. burgdorferi sensu lato* in a sample of human or animal fluid which method comprises the steps of
(i) contacting any sample antibodies to be detected with anti-IgM immobilised to a solid support,
(ii) optionally separating the solid support from the liquid phase,
(iii) contacting the antibodies bound to the solid support with an antigen complex comprising a carrier to which is attached one or more sets of *Borrelia* OspC peptides, and an antigen complex comprising a carrier to which is attached one or more sets of other *Borrelia* peptides, each set comprising a plurality of *Borrelia* peptides representing the same antibody binding epitope and binding to IgM molecules, which antigen complex is or will be labelled, and
(iv) determining the presence, if any, of antibodies against *B. burgdorferi sensu lato.*

In further aspects, the present invention relates to a method for the determination of IgM antibodies against *B. burgdorferi sensu lato* in a sample of human or animal fluid which method comprises the steps of
(i) contacting any sample antibodies to be detected with anti-IgM immobilised to a solid support,
(ii) separating the solid support from the liquid phase,
(iii) contacting the antibodies bound to the solid support with an antigen complex comprising a carrier to which is attached one or more sets of *Borrelia* OspC peptides and optionally one or more sets of other *Borrelia* peptides, each set comprising a plurality of *Borrelia* peptides representing the same antibody binding epitope and binding to IgM molecules, which antigen complex is or will be labelled, and
(iv) determining the presence, if any, of antibodies against *B. burgdorferi sensu lato,*
and to a method for the determination of IgM antibodies against *B. burgdorferi sensu lato* in a sample of human or animal fluid which method comprises the steps of
(i) contacting any sample antibodies to be detected with anti-IgM immobilised to a solid support,
(ii) separating the solid support from the liquid phase,
(iii) contacting the antibodies bound to the solid support with an antigen complex comprising a carrier to which is attached one or more sets of *Borrelia* OspC peptides, and an antigen complex comprising a carrier to which is attached one or more sets of other *Borrelia* peptides, each set comprising a plurality of *Borrelia* peptides representing the same antibody binding epitope and binding to IgM molecules, which antigen complex is or will be labelled, and
(iv) determining the presence, if any, of antibodies against *B. burgdorferi sensu lato.*
To the carrier is attached one or more sets of *Borrelia* OspC peptides, each set comprising a plurality of *Borrelia* peptides, each set representing the same antibody binding epitope and binding to IgM molecules. The sets of peptides can be derived from one or more species of *B. burgdorferi sensu lato.* The antigen complex may further comprise one or more sets of peptides derived from other *Borrelia* proteins. The peptides of a set will typical be identical. For detecting the early stages of Lyme borreliosis, the antigen complex may e.g. comprise peptides derived from *Borrelia* flagellin.

In one aspect of the present method, the antibodies to be detected are in step (iii) contacted with a labelled antigen complex comprising a carrier to which is attached one or more sets of *Borrelia* OspC peptides, each set being represented by a plurality of identical peptides, and in which method non-bound antigen complexes are removed before step (iv).

The carrier of the antigen complex may be a solid or semisolid carrier such as a particle, or a microsphere, or it may be an insoluble or soluble organic or inorganic polymer, the *Borrelia* flagellum or a fragment thereof or it may be a combination of a carrier and a further component selected from insoluble or soluble organic or inorganic polymers, the *Borrelia* flagellum or a fragment thereof. Whole cells may also be used as carriers.

It is preferred to use a labelled antigen complex, wherein the label is attached either directly to the carrier by covalent bonds or covalently to streptavidin, avidin or a functional derivative thereof which again is attached to the carrier through biotin.

The *Borrelia* peptides may be attached covalently to the carrier or the antigen complex may be obtained by biotinylating the peptides and the carrier, allowing the peptides and the carrier to complex through streptavidin, avidin or a functional derivative thereof.

In a preferred aspect, the present invention relates to a method wherein the carrier of the antigen complex is the *Borrelia* flagellum or fragments thereof to which is attached a plurality of identical *Borrelia* OspC peptides, the flagellum or fragments thereof and the OspC peptides being biotinylated and attached to each other through streptavidin, avidin or a functional derivative thereof to which an enzyme is covalently attached.

In yet another preferred aspect, the present invention relates to a method, wherein the carrier is a combination of a solid or semisolid carrier and the *Borrelia* flagellum or fragments thereof, the carrier components being biotinylated and attached to each other through streptavidin to which an enzyme is covalently attached and to which particulate carrier is attached a plurality of identical *Borrelia* OspC peptides.

Other aspects of the invention are the antigen complex and kits comprising said complex.

### BRIEF DESCRIPTION OF THE DRAWINGS

Fig. 1 shows the reaction of human serum samples in different *anti-Borrelia* IgM assays. Serum sample nos. 311, 7696, 106/65, 331/48 and 3959/59 are from patients with a clinical diagnosis of Lyme borreliosis, and serum sample nos. 440, 441 and 442 are from Danish blood donors. Abbreviations: conseq., consequtively; equiv., equivocal; neg., negative; n.t., not tested; OD, optical density (reaction in ELISA); pos., positive. Index is the actual sample OD divided by the assay cut-off control OD.

Fig. 2 shows titration of OspC/biotin (seq. no. 2) against streptavidin/HRP and subsequent complexing with biotinylated flagella: Influence of the OspC/biotin concentration on the reaction of the antigen complex with different human serum samples.

Fig. 3 shows titration of OspC/biotin (seq. no. 1) against streptavidin/HRP and subsequent complexing with biotinylated flagella: Influence of the OspC/biotin concentration on the reaction of the antigen complex with different human serum samples.

Fig. 4 shows titration of biotin and OspC (seq. no. 3) conjugated latex beads against streptavidin/HRP: Influence of the latex bead density on the reaction of the antigen complex with different human serum samples.

Fig. 5 shows titration of biotin and OspC (seq. no. 3) conjugated latex beads against streptavidin/HRP and subsequent complexing with biotinylated flagella: Influence of the latex bead density on the reaction of the antigen complex with different human serum samples.

Fig. 6 shows the index value (sample OD / cut-off OD) of 103 blood donor sera as obtained in the assay according to the invention relative to that obtained with the IDEIA™ *Borrelia burgdorferi* IgM (DAKO cat. no. K 6030). The cut-off index value of 1 is shown by dashed lines.

Fig. 7 shows the index value (sample OD / cut-off OD) of 27 sera from patients with a clinically observed *erythema migrans* obtained in the assay according to the invention relative to that obtained with the IDEIA™ Borrelia burgdorferi IgM (DAKO cat. no. K 6030). The cut-off index value of 1 is shown by dashed lines.

Fig. 8 shows the index value (sample OD / cut-off OD) of 96 sera from patients with a neuroborreliosis obtained in the assay according to the invention relative to that obtained with the IDEIA™ *Borrelia burgdorferi* IgM (DAKO cat. no. K 6030). The cut-off index value of 1 is shown by dashed lines.

### DETAILED DESCRIPTION OF THE INVENTION

The antigen complex of the invention provides an improvement in the diagnosis of Lyme borreliosis. In particular, the simultaneous detection of antibodies against the two important early immunodominent *Borrelia* antigens, OspC and the flagellum, has provided a labour and cost saving method that is well suited for routine testing.

In the following, a number of terms will be explained in greater detail.

By the term "antigen complex" is herein meant a chemical entity which at least is capable of reacting with antibodies specific for OspC of *Borrelia burgdorferi sensu lato.* The complex comprises
a carrier to which is attached one or more sets of *Borrelia* OspC peptides and optionally one or more sets of other *Borrelia* peptides, each set comprising a plurality of *Borrelia* peptides representing the same antibody binding epitope and binding to IgM molecules, or
a carrier to which is attached one or more sets of *Borrelia* OspC peptides and a carrier to which is attached one or more sets of other *Borrelia* peptides, each set comprising a plurality of *Borrelia* peptides representing the same antibody binding epitope and binding to IgM molecules.

The complex is preferably labelled but it may also be contacted with the antibodies to be detected in unlabelled form followed by detection of the specific immunological reaction by addition of a labelled detection reagent such as a labelled Fab fragment of anti-human IgM or a labelled Fab fragment reacting with the antigen of the antigen complex.

The term *"Borrelia burgdorferi sensu lato"* covers *B. burgdorferi sensu stricto, B. garinii, B. afzelii* and *B. valaisiana.*

The terms *"Borrelia* OspC peptides" and "other *Borrelia* peptides" do in the present context mean a sequence of amino acid residues that can be identical to a sequence of a native *Borrelia* protein or be substantially similar to such a sequence, e.g. differing by one or a few amino acids, with the proviso that the peptides are able to react with antibodies specific to *Borrelia.* The "other *Borrelia* peptides" may be derived from other *Borrelia* proteins eliciting a response in the majority of Lyme borreliosis patients. For detecting an early response, other *Borrelia* peptides may be derived from *Borrelia* flagellin (p41), the outer surface proteins E or F.

It is preferred that the amino acid sequence of the peptides share at least 80% homology with the native sequence when the peptide is composed of 10 or less amino acids. When the peptide is composed of more than 10 amino acids, it is preferred that the sequence shares at least 60% homology with the native sequence.

The length of the peptides is preferably at most 60 amino acids, more preferably at most 30 amino acids, such as from 2 to 30 amino acids, preferably from 4 to 30 amino acids, more preferably from 4 to 25 amino acids such as from 5 to 25. In a preferred embodiment, the length of the peptides is less than 10 amino acids, such as from 2 to 9 amino acids, suitably from 4 to 9 amino acids such as 7 or 8 amino acids.

In ref. 3, the 4 C-terminal amino acids of OspC have been shown to be very important for binding when testing for antibodies against *B. burgdorferi sensu lato* OspC. Thus, in a special embodiment of the present invention, the 4 C-terminal amino acids should be included in the OspC peptides of the present antigen complex. Peptides having a length of from 4 to 30 amino acid residues counting from the C-terminal end of OspC are preferred, the most preferred having a length of from 4 to 25 amino acids, or from 5 to 25 amino acids, in particular less than 10 amino acids, preferably from 4 to 9 amino acids, or from 5 to 9 amino acids such as 7 or 8 amino acids. Suitable amino acid sequences of OspC are disclosed in ref. 3.

The carrier is such to which one or more sets of peptides, each set comprising a plurality of peptides representing the same antibody binding epitope and binding to IgM molecules, are attached, thereby presenting multiple binding sites for the peptid-specific IgM antibodies within the same complex. As defined above, the carrier may be a solid or semisolid carrier. Non-limiting examples are particles such as a latex particles, microspheres such as metal particles, and whole cells such as erythrocytes. Or the carrier may be an insoluble or soluble organic or inorganic polymer, the *Borrelia* flagellum or a fragment thereof. Furthermore, the carrier may be a combination of such carriers such as a combination of particles or microspheres and insoluble or soluble organic or inorganic polymers, the *Borrelia* flagellum or fragments thereof.

By the term "a plurality" is herein meant a number of peptides that is sufficiently large and arranged in such a way that multipoint attachment to one or more captured IgM antibodies is achieved thereby securing the antibody against being lost during the subsequent steps. The minimum number of peptides is likely to differ depending on several factors, i.a. on the nature of the carrier as well as on the assay format. The number of peptides available on a carrier may, depending on the size and type of carrier be very large, up to or more than 100.000 or even up to or more than 1.000.000. In other cases, as few as 2-10 or 2-1000 peptides may be sufficient.

In the present context, the term "the antigen complex is or will be labelled" relates to the two possibilities that the antigen complex carries a label or is unlabelled when brought in contact with the antibodies to be detected. In the latter case it is subsequently labelled by addition of a labelled secondary binding partner such as a labelled Fab fragment of anti-human IgM. In order to avoid interference from RF, a labelled IgG antibody should not be used. The term " the antigen complex is labelled" includes complexes between a *per se* unlabelled antigen complex and a labelled secondary binding partner if the complexes are formed prior to contact with the antibody to be detected. Examples of labelled secondary binding partners are streptavidin/HRP and anti-biotin/HRP. Others can of course be used and are well-known to the person skilled in the art.

When the term "streptavidin" is mentioned hereinafter, it should be contemplated that streptavidin can be replaced by avidin or derivatives of streptavidin or avidin having a biotin-binding capacity.

The method according to the present invention is preferably performed in microwells coated with antibodies specific to IgM but may also be performed using another solid support. Other suitable solid supports are microspheres such as paramagnetic particles implying magnetic separation of bound and non-bound components. Yet another suitable solid supports are polystyrene beads and membranes. Furthermore, the method may be adapted to a flow cytometry test if e.g. the IgM catching antibody is immobilised onto suitable particles or whole cells.

The antibodies specific to IgM may be immobilised to the solid support by adsorption or may in biotinylated form be added to a streptavidin coated solid support. The sample, which is preferably serum or cerebrospinal fluid, is then contacted with the immobilised antibodies for binding of IgM antibodies of various specificity. A proportional fraction of any RF of the IgM class in the sample will also be immobilised. Non-bound components of the sample are removed by separating the solid support from the liquid phase, e.g. by washing the solid support. This separation step is essential to remove unbound antibodies against *Borrelia* and to avoid interference related to any RF bound to the solid support.

In one embodiment, the antibodies bound to the solid support are brought into contact with an antigen complex comprising one or more sets of *Borrelia* OspC peptides and optionally one or more sets of other *Borrelia* peptides, each set comprising a plurality of *Borrelia* peptides representing the same antibody binding epitope and binding to IgM molecules. Suitable OspC peptides are described in WO 97/42221. The design of the antigen complex allows multivalent binding to the IgM antibodies bound to the solid support resulting in an increased stability of the binding between the antigen complex and IgM antibodies reacting with the peptides of the antigen complex.

The antigen complex is preferably labelled with a detectable moiety. The detectable moiety may be selected from the group consisting of chromophore, fluorochrome, spin label, radioisotope, enzyme, hapten and chemiluminescent compounds. Examples of suitable enzymes are horseradish peroxidase, alkaline phosphatase and soybean peroxidase. Non-limiting examples of haptens are fluorescein, biotin, 2,4-dinitrophenyl and digoxigenin. The labelling procedure can be carried out using conventional methods known to the person skilled in the art.

Both the peptides and the detectable moiety may be attached covalently to the carrier using a homobifunctional coupling reagent such as glutaraldehyde or a heterobifunctional coupling reagent such as SPDP (3-(2-pyridyldithio)propionic acid N-hydroxysuccinimide ester) or SMCC (4-(N-maleimidomethyl)-cyclohexane-1-carboxylic acid 3-sulfo-N-hydroxysuccinimide ester). Furthermore, the peptides and the carrier may be biotinylated and attached to each other through streptavidin. The detectable moiety may be covalently attached to streptavidin which is subsequently attached to the carrier through biotin. To avoid sterical problems, a linker may be introduced between the carrier and the peptide, biotin or another detectable moiety. Non-limiting examples of suitable linkers are one or more units of [2-aminoethoxy]ethoxy acetic acid (O-linker) or ethylene oxide. Furthermore, an amino acid can be used as a linker. The amino acid cystein may e.g. be used to provide a SH group to be used in the coupling reaction.

Depending on the method used in step (iv) for determining the presence of antibodies against *Borrelia burgdorferi sensu lato,* separation of the solid support from the liquid phase prior to step (iv) may or may not be necessary. If the solid support is particles for analysis in flow cytometry, such a separation step is not necessary, but in a format using microwells, non-bound antigen complexes should be removed.

In a preferred embodiment of the method according to the invention, the carrier of the antigen complex is the *Borrelia* flagellum or fragments thereof. The *Borrelia* peptides may be attached to the *Borrelia* flagellum or a fragment thereof, or the *Borrelia* peptides may be attached to a carrier which again is attached to the *Borrelia* flagellum or a fragment thereof. The combined function of the flagellum as a carrier and as contributing relevant epitopes for detection of IgM antibodies against the flagellum allows for the simultaneous determination of antibodies against *Borrelia* OspC and the *Borrelia* flagellum.

Another aspect of the invention is the antigen complex as defined in relation to the method described above. If the antigen complex is labelled with a detectable moiety, which can de detected directly such as a fluorescent or radioactive moiety, no further reagents are needed in step (iv). In other cases, means for detection/or measuring the immunological reaction may be needed.

Hence, a further aspect of the invention is a kit comprising a labelled antigen complex as defined above wherein the label is not directly detectable and a substrate for visualising the immunological reaction.

Yet another aspect is a kit comprising an unlabelled antigen complex as defined above, a labelled secondary binding partner and optionally a substrate for visualising the immunological reaction.

A preferred kit comprises an antigen complex wherein the carrier is *Borrelia* flagellum or fragments thereof to which is attached a plurality of identical *Borrelia* OspC peptides, the flagellum or fragments thereof and the OspC peptides being biotinylated and attached to each other through streptavidin to which an enzyme is covalently attached.

Another preferred kit comprises an antigen complex wherein the carrier is a combination of a particulate carrier and the *Borrelia* flagellum or fragments thereof, the carrier components being biotinylated and attached to each other through streptavidin to which an enzyme is covalently attached, to which particulate carrier is attached a plurality of identical peptides.

The complexes described herein can be produced in a highly stable form maintaining high levels of antigenicity. The complexes produced can be freeze-dried and reconstituted in a suitable buffer retaining a high level of activity and is thus a very suitable reagent component for use in diagnosing Lyme borreliosis.

The invention is further illustrated by the non-limiting examples given below.

### EXAMPLES

### EXAMPLE 1

### Preparation of antigens

### A. Preparation of Borrelia peptides and optional biotinylation thereof.

The *Borrelia* peptides were synthesised by the solid phase 9-fluorenylmethoxy-carbonyl (Fmoc) method (ref. 4). The solid-phase peptide synthesis was performed using a multi-peptide synthesiser AMS422 (Abimed, Langenfeld, Germany) as described in Gausepohl et al (1990). All peptides were synthesised with Fmoc amino acids (Novabiochem) using PyBOP (benzotriazole-1-yloxytripyrrolidinophosphonium hexafluorophosphate, Novabiochem) as coupling reagent. The side-chain protection was as follows: Asn, Gln and His (trityl); Arg (2,2,5,7,8-pentamethylchroman-6-sulfonyl); Asp, Glu, Tyr, Ser and Thr (*tert*-Butyl); Trp and Lys (*tert*-butyloxycarbonyl).

0.7 M solutions in dimethylformamide or N-methyl pyrrolidone of the amino acids (4 times excess) containing equimolar amounts of PyBOP and 2 equivalents of N-methylmorpholine (Fluka) were used for the couplings. An acid-labile H-Pro-2-cltrt resin (Novabiochem) was used for preparation of C-terminal proline-containing peptide carboxylates.

Biotinylated peptides were prepared after the assembly of the peptide chain. The alfa N Fmoc protecting group was removed and one or two of Fmoc protected O-linkers (N-(9-fluorenylmethoxycarbonyl) [2-aminoethoxy] ethoxy acetic acid, PerSeptive Biosystem) were coupled to the N-terminal of the peptide using HATU (*N*-[(dimethylamino)-1H-1,2,4-triazol [4,5-b]pyridine-1-yl-methylene]-*N*-methylmethanaminium hexafluorophosphate N-oxide, PerSeptive Biosystem) and HOAT (1-hydroxy-7-azabenzotriazole, PerSeptive Biosystem) as coupling reagent. Biotinylation was done with biotin and HATU and HOAT.

For covalent coupling of peptides to latex particles a N-terminal cystein residue was incorporated providing a SH group to be used in the coupling reaction.

The peptides were cleaved from the resin with trifluoroacetic acid/triethylsilane/H₂O 92.5:5:2.5 v/v/v at room temperature (21-23°C) for 2 h. The peptides were precipitated in methyl tertbutyl ether and washed with ether and dried in vacuum. The peptides were purified by HPLC (Vydac 218TP1022, flow rate 9 ml/min) using a gradient of water and acetonitril, both containing 0.1 % trifluoroacetic acid. All peptides were more than 90% pure. Identity of the peptide was confirmed by MALDI-TOF mass spectrometry using an HP G2025A instrument.

The following *Borrelia* OspC peptides were synthesised as described above:
Biotin-L-L-Val Ala Glu Ser Pro Lys Lys Pro-COOH (seq. no 1), wherein each L is an O-linker of the formula [2-aminoethoxy]ethoxy acetic acid.
Biotin-L-Gln Val Ala Leu Thr Asn Ser Val Lys Glu Leu Thr Ser Pro Val Val Ala Glu Ser Pro Lys Lys Pro-COOH (seq. no 2), wherein L is as defined above.

Cystein-Gln Val Ala Leu Thr Asn Ser Val Lys Glu Leu Thr Ser Pro Val Val Ala Glu Ser Pro Lys Lys Pro-COOH (Seq. No 3), wherein the N-terminal cystein residue has been incorporated to provide a SH group to be used in the coupling reaction.

### B. Preparation of native Borrelia flagella and optional biotinylation thereof.

The flagella of *in vitro*-grown *B. afzelii* strain DK1 were isolated, purified and optional biotinylated as described in ref. 1 and 2.

Biotinamidocaproate N-hydroxysuccinimide ester, 20 mM in dimethylformamide, was used to covalently biotinylate the flagella in a ratio of 0.14 mg biotin reagent to 1 mg of flagellum protein. The reaction took place in 0.125 M NaHCO₃ buffer pH 9.0 for 8 h at room temperature, after which the reaction mixture was dialysed against PBS for at least 24 h. The dialysate was the final preparation of purified, biotinylated *Borrelia* flagella.

### EXAMPLE 2

### Preparation of antigen complexes according to the invention

### A. By covalent coupling of Borrelia OspC peptides and biotin to particles and subsequent reaction with streptavidin/HRP conjugate and optionally biotinylated Borrelia flagella.:

Purification of carboxyl modified latex (CML):
15.0 ml of CML (Seradyn Inc., art. no. 13000350100490, lot 2061, 10.0 % by weight, average diameter of 107 nm, 0.1566 mmol carboxyl groups per gram) was mixed with ion exchange resin (3.0 gram, mixed bed resin, BioRad Laboratories) and shaken for 2 h. The mixed bed resin was removed by filtration (MiniSart, 5 µm, Sartorious AG, no. 175-94-K).

Pre-activation of CML and coupling of polyethylene glycol (PEG) linker to form NH₂-PEG-CML:
To a suspension of 5.0 ml purified CML (10 % latex) was added 3.0 ml 0.10 M N-morpholinoethanesulfonic acid (MES) buffer (Sigma Chemical Co., pH 6.0), a solution of sulfo N-hydroxysuccinimide (1.0 ml, 158 mM, Pierce Chemical Company, in 0.10 M MES, pH 6.0), and a solution of 1-ethyl 3-(dimethylaminopropyl)carbodiimide hydrochloride (1.0 ml, 40 mM, Sigma Chemical Co., in 0.10 M MES, pH 6.0. The suspension was tip sonicated (75 µA, 60 seconds, 0°C) using a Labsonic 2000, ultrasound generator, B-Braun, and shaken for 20 minutes at room temperature.

The sulfo N-hydroxy succinimide ester activated latex suspension was slowly added to a magnetically stirred O,O'-Bis(2-amino propyl) ethylene glycol 1900 solution (10.0 ml, 20%, Fluka AG, approx. Mᵣ 2000 g/mol, in 0.10 M MES, pH 6,0) while being continuously tip sonicated (75 µA) and cooled on a ice-bath. After stirring for 2 h at room temperature, 15 ml water was added to the suspension. Twice the following procedure was carried out, the modified latex was isolated by centrifugation (20,000 x g, 3 h, 4°C), the pellet re-suspended in water (15 ml) and tip sonicated (75 µA, 3 times 45 seconds, 0°C).

The amino propyl polyethylene glycol modified latex suspension was mixed with ion exchange resin (3.0 g, BioRad Laboratories) and shaken for 2 h. The mixed bed resin was removed by filtration (MiniSart, 5 µm). The amino-PEG modified latex was isolated by centrifugation (20,000 x g, 3 h, 4°C). The pellet was re-suspended in water (7.50 ml) and stored in the cold (4°C).

A sample was qualitatively positive for free primary amino groups as determined by Kaiser Ninhydrine test.

Coupling of biotin and peptide to NH₂-PEG-CML to form Biotin-PEG-CML-PEG-OspC:
The amino propyl polyethylene glycol modified latex (1000 µl, approx. 0.66 % latex) was isolated by centrifugation (20,000 x g, 1.5 h, 4°C) re-suspended in MES buffer (875 µl 0.10 M, pH 6.0). To this suspension was added N-hydroxy succinimide biotin solution (125 µl 42.1 mM, Sigma Chemical Co., in dimethylsufoxide, Aldrich Chemical Company).

After 1 h at room temperature, the modified latex was isolated by centrifugation (20,000 x g, 1.5 h, 4°C). The pellet was re-suspended in MES buffer (900 µl, 0.10 M, pH 6.0) and to this suspension was added a solution of succinimidyl-4-(N-maleimidomethyl)-cyclohexan-1-carboxylate (100 ml, 112.2 mM, Pierce Chemical Comp., in dimethylsulfoxide). The suspension was incubated for 1 h at room temperature followed by 18 hours at 4°C.

The biotin and maleinimide modified latex was isolated by centrifugation (20,000 x g, 1.5 h, 4°C). The pellet was washed by re-suspension in a potassium phosphate buffer (1000 µl, 40 mM potassium phosphate (E.Merck), 2 mM EDTA (Sigma Chemical Co.), 300 mM NaCl (E.Merck), pH 7.2), followed by latex isolation by centrifugation (20,000 x g, 1.5 h, 4 °C) and resuspension in potassium phosphate buffer (800 µl, 40 mM potassium phosphate, 2 mM EDTA, 300 mM NaCl, pH 7.2).

To the biotin and maleinimide modified latex (400 µl) was added a solution of synthetic OspC peptide (100 µl of a solution of 8,9 mg lyophilised Cystein-Gln Val Ala Leu Thr Asn Ser Val Lys Glu Leu Thr Ser Pro Val Val Ala Glu Ser Pro Lys Lys Pro-COOH (seq no 3), lot OK-0568-03, in 300 µl 40 mM potassium phosphate, 2 mM EDTA, 300 mM NaCl, pH 7.2). The suspension was incubated for 3 h at room temperature before a cystein solution (100 µl, 0.825 M, Sigma Chemical Co., in 40 mM potassium phosphate, 2 mM EDTA, 300 mM NaCl, pH 7.2) was added. The modified latex was washed four times by first being isolated by centrifugation (20,000 x g, 1.5 h, 4°C), re-suspended in a potassium phosphate Tween 20 buffer (1000 µl, 0.10 M KP, 0.50% Tween 20 (E. Merck) pH 7.2) and tip sonicated (75 µA, 3 times 45 seconds, 0°C). After washing, the biotin and peptide modified latex was isolated by centrifugation (20,000 x g, 1.5 h, 4 °C). The pellet was re-suspended in a potassium phosphate Tween 20 buffer (500 µl, 0.10 M potassium phosphate, 0.50% Tween 20, pH 7,2) and stored cold (4°C). As will appear, biotin and OspC peptides are covalently attached to different sites of the particles.

To obtain biotinylated latex particles to which is coupled OspC peptides as well as an enzyme label, particles modified as described above were preincubated with a streptavidin/HRP conjugate.

In another embodiment of the present antigen complex, biotinylated latex particles to which is attached OspC peptides are pre-incubated with streptavidin/HRP conjugate and subsequently with biotinylated flagellum.

### B. By binding biotinylated Borrelia flagellum and OspC peptides together through streptavidin/HRP conjugate

Throughout this experiment, biotinylated flagella and streptavividin/HRP were taken from the Lyme borreliosis IgM ELISA kit produced by DAKO (Denmark, IDEIA™ *Borrelia burgdorferi,* IgM, cat. no. K 6030) and used in exactly the form and manner specified in the product manual. The assay principle and procedure is outlined below in Example 3, section A.

In order to allow co-complexing of biotinylated peptides and biotinylated flagellum with a streptavidin/HRP conjugate and to avoid any deleterious competition between the flagellum and the peptides in their binding to the streptavidin/HRP conjugate, it was necessary to determine the optimal stoichiometry between the three partners in the complex. The experiment involved two complexing steps. In the first step, OspC peptide/biotin, previously dissolved in destilled water or buffer to a known molarity, and a solution of streptavidin/HRP conjugate of specified streptavidin molarity, were mixed in increasing, i.e. 0 to 64, stoichiometric ratios of biotinylated peptide to streptavidin. The concentration of streptavidin/HRP was kept constant, whereas that of the biotinylated OspC peptide was varied according the desired ratio of biotin to streptavidin. The two compounds were allowed to react during a one-hour incubation period at room temperature. In the second complexing step, a volume of the peptide/biotin - streptavidin/HRP complex was added to a volume of biotinylated flagella of known concentration. The two volumes were predetermined such as to involve all biotinylated flagella and all streptavidin/HRP molecules in complexing in the case of absence of biotinylated peptide. Those volumes were kept constant, irrespectively of the amount of biotinylated peptide added during the first step to the streptavidin/HRP. The reaction during the second complexing step was allowed to take place during an one hour incubation at room temperature.

In the first complexing step the chosen stoichiometric ratios of biotinylated peptide to streptavidin were 0, 1, 1.5, 2, 2.5, 3, 4, 8,16, 32, and 64, respectively. From a) knowing the stoichiometric ratio and b) the fact that streptavidin has four biotin-binding sites per molecule, the number of biotinylated peptide molecules bound per streptavidin molecule could be approximated, as could the relative amount of unbound, or surplus, biotinylated peptide. In this manner the biotinylated flagella's possibility of being part of the complex could be estimated.

The performance of the various complexes prepared in A and B were tested in a µ-capture assay described below.

### EXAMPLE 3

### µ-capture ELISA using different antigen complexes

### A. Essentials about the assay and serum samples applied

Eight human serum samples were used for some experiments. Three sera (nos. 440, 441, 442) were from presumably healthy Danish blood donors. Five sera (nos. 311, 7696, 106/65, 3311/48, 3959/59) were obtained from patients with a clinical diagnosis of Lyme Borreliosis and were chosen as to represent different combinations of presence/absence of anti-flagellum and anti-OspC IgM.

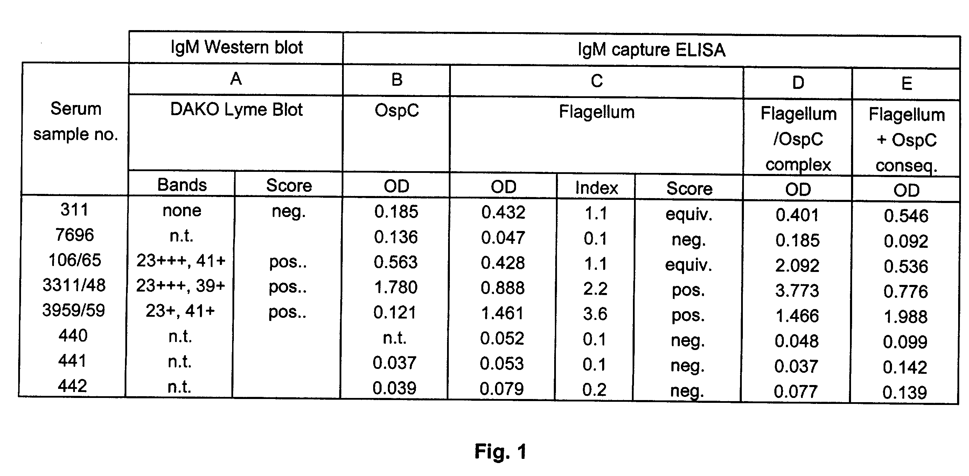

Data applying to four of the patient sera was available from testing in the DAKO Lyme Blot IgM immuno-Western blot assay (DAKO, Denmark, cat. no. K 0120), which shows the presence of native *B. burgdorferi* proteins, including flagellin (p41) and OspC (p23) (Fig. 1, column A).

A panel of 103 randomly collected sera from Norwegian blood donors was used to assess the diagnostic cut-off level of the ELISAs.

A panel of sera from 27 Swedish patients with a clinical diagnosis of *erythema migrans* and a panel of 96 sera taken from Danish patients and submitted for laboratory diagnosis of neuroborreliosis were used to compare the diagnostic performance of ELISAs.

Wherever tested in ELISA, the sera were diluted 1+200 prior to use, such as described below.

The enzyme immunoassay used throughout in the experiments was the Lyme borreliosis IgM ELISA kit produced by DAKO (Denmark, IDEIA™ *Borrelia burgdorferi,* IgM, cat. no. K 6030). In its original form, the assay principle and steps are as follows: The kit contains microtiter plates with wells coated with rabbit anti-human IgM. The first step is pipetting 100 µl/well of diluted human serum samples. IgM present in the sample is captured during the first incubation, which is carried out at room temperature for 1 h on an orbital shaker at 500 rpm. Then the wells are washed four times with a buffer solution to remove unbound serum proteins, followed by addition of 100 µl/well of native biotinylated *B. burgdorferi* flagella complexed with streptavidin/HRP conjugate to each well. The complex has been pre-formed by the user by reconstitution of lyophilised, biotinylated flagella in buffer, followed by addition of a volume of streptavidin/HRP. The complex binds only to *B. burgdorferi* flagellum-specific IgM antibodies. Following this second incubation step, which is also carried out for 1 h by room temperature, excess complex is removed by washing the microtiter wells four times, and following addition of 100 µl/well of an HRP substrate solution (tetramethylbenzidine; hydrogen peroxide) the bound enzyme catalyses the development of a blue colour. The enzyme reaction is stopped by addition of 100 µl/well of dilute sulphuric acid, which changes the blue colour to yellow. The colour intensity is determined spectrophotometrically at 450 nm and the absorbance value of each well is compared with the absorbance values of controls. The "cut-off value" was chosen to exclude 98% of sera from healthy donors from being evaluated as containing anti-*B. burgdorferi* IgM antibodies. The actual value corresponds to the concentration of *B. burgdorferi* flagellum-specific IgM antibodies in the tested serum sample.

### B. Comparison of different antigen complexes

### a. Prior art complex containing biotinylated flagella and streptavidin/HRP

The Lyme borreliosis IgM ELISA kit produced by DAKO (Denmark, IDEIA™ *Borrelia burgdorferi,* IgM, cat. no. K 6030) implies the use of biotinylated flagella complexed with streptavidin/HRP, which is regarded as prior art antigen complexes in the present context. Results obtained by testing the sera in this assay are listed in Fig. 1, column C. It will be seen that among the patient sera, one was negative (7696), two were equivocal (311, 106/65), one was weakly positive (3311/48), and one strongly positive (3959/59) for anti-flagellum IgM. The three blood donor sera (440, 441, 442) were negative.

### b. Complex containing biotinylated OspC peptide and streptavidin/HRP

The IDEIA™ *Borrelia burgdorferi,* IgM, cat. no. K 6030 was used, the only change being that OspC peptide seq. no. 2 was substituted for the biotinylated flagellum (see Example 1, Section A). Prior to addition to the wells, the OspC peptide/biotin and the streptavidin/HRP were allowed to complex, the peptide concentration being adjusted so as to yield a biotin to streptavidin ratio of 8:1. The results listed in Fig. 1, column B indicate that three patient samples were negative for anti-OspC IgM (311, 7696, 3959/59), one equivocal (106/65), and one positive (3311/48). The three blood donor sera were negative.

### c. Antigen complexes according to the invention

Again the IDEIA™ *Borrelia burgdorferi,* IgM, cat. no. K 6030 was used, the only change being that antigen complexes according to the invention were substituted for the flagellum/biotin-streptavidin/HRP complex.

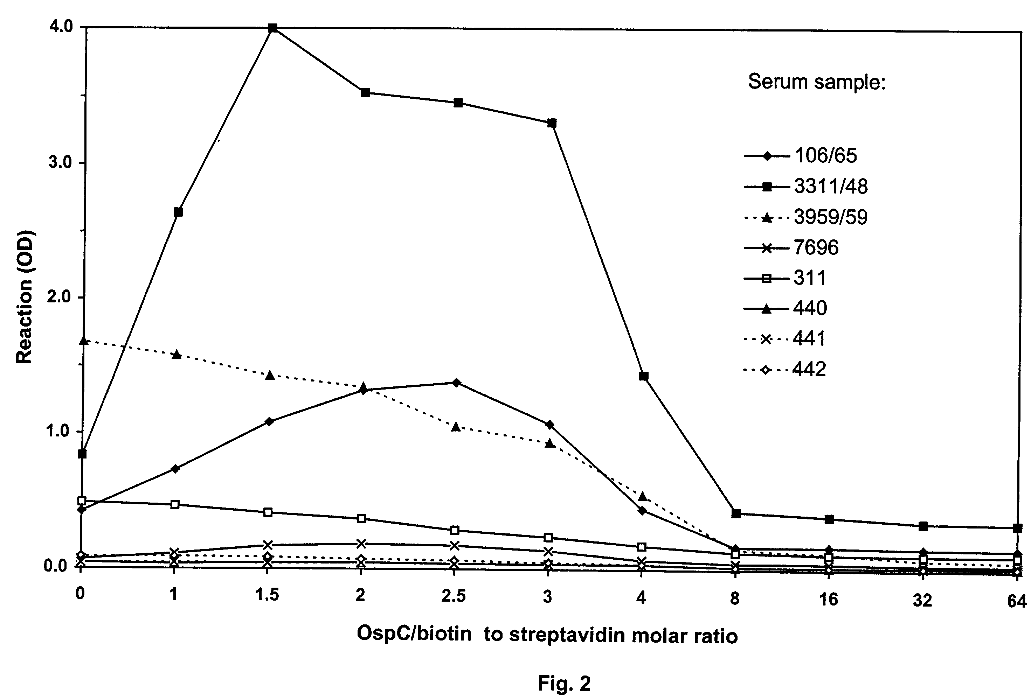

Firstly, the optimal ratio between biotinylated flagella, biotinylated OspC peptide and streptavidin/HRP was determined, such as described above (see Example 2, Section B). Stoichiometric ratios of biotinylated OspC peptide seq. no. 2 (see Example 1, Section A) to streptavidin ranging from 0 to 64 were tested. The results of this experiment are shown in Figure 2. It will be understood that at a ratio of zero, no OspC peptide was present in the complex which therefore contained biotinylated flagellum and streptavidin/HRP only. Because the assay thereby in fact was the IDEIA™ *Borrelia* *burgdorferi,* IgM, the results shown closely resemble those listed in Fig. 1, column D and described in Example 3, Section B, Paragraph a.

A ratio of 1 or 2 allows for the anticipation that on average one or two peptide molecules are bound to each streptavidin, leaving three or two biotin-binding sites per streptavidin for reaction with biotins conjugated to flagella and resulting in an antigen complex according to the invention, i.e. an OspC peptide/biotin - flagellum/biotin - streptavidin/HRP complex.

A ratio of 4 means that the molecular amount of OspC peptide/biotin is four times the molecular amount of streptavidin. Because OspC peptide/biotin and streptavidin/HRP are allowed to complex prior to the addition of biotinylated flagella, most complexes will consist of OspC peptide/biotin and streptavidin/HRP only, and very few will be formed that contain biotinylated flagella as well.

A ratio of 8, 16, 32 or 64 means that OspC peptide/biotin is allowed to occupy all biotin-binding sites on the streptavidin, resulting in complexes practically devoid of flagella.

The most important finding is that when serum samples expected to contain antibodies to OspC are tested (Fig. 1, column A; Fig. 2, curves for sera 106/65, 3311/48), maximum assay signals are obtained when the flagellum/biotin forms part of the complex, i.e. with an OspC peptide/biotin to streptavidin ratio < 4 and > 0 (Fig. 1, column D), whereas they decrease significantly when the flagellum/biotin is precluded from forming part of the complex by the occupation of all biotin-binding sites on the streptavidin by peptide/biotin, i.e. with OspC peptide/biotin to streptavidin ratios > 4 (Fig. 1, column B). Without being bound by the theory, the most obvious reason for this finding relates to the concerted effect of a) the number of biotin molecules per peptide and flagellum, b) the spacing between peptides held by complex, and c) the low avidity of the IgM antibody class. Each peptide has one biotin only, whereas biotinylation of purified preparations of flagella leaves numerous biotins per flagellum. In the case of an OspC peptide/biotin to streptavidin ratio > 4 there is only a single streptavidin/HRP conjugate per complex, and hence the peptides bound in each complex are situated as close together as depicted by the spacing between the biotin-binding sites on the streptavidin. This may imply that each complex is able to bind to only one of the anti-OspC IgM antibodies captured on the solid support. Due to the low avidity of the IgM, the complex is prone to be released during the subsequent washing of microtiter wells, thereby decreasing the sensitivity of the assay.

On the contrary, when the peptide/biotin to streptavidin ratio is < 4 and > 0, the flagellum/biotin is incorporated with OspC peptide/biotin in each complex, which again contains several streptavidin/HRPs. Such complexes as herein defined may be pictured as several peptide/biotin - streptavidin/HRP subcomplexes held together by a flagellum/biotin backbone, making the binding of the whole complex to several anti-OspC IgM antibodies possible. In this way the complex is much less prone to be washed off from anti-OspC IgM captured on the solid support during the first incubation step.

To exclude that the effect obtained is mainly an amplification, the following assay was carried out. The incubation with serum was followed by incubation with OspC/biotin, and then in a separate step with flagellum/biotin and streptavidin/HRP (Fig. 1, column E; conseq.). The signal obtained is similar to the signal obtained with anti-flagellum antibodies only (Fig 1., column C).

A second finding is that detection of anti-flagellum IgM is retained even if OspC peptide/biotin is included besides flagellum/biotin in the complex (Fig. 2, curves for sera 3959/59 and 311; Fig. 1, column D). Obviously, the detection is completely hindered when the OspC peptide/biotin to streptavidin ratio > 4, i.e. when biotinylated peptide is allowed to occupy all biotin-binding sites on the streptavidin, resulting in complexes devoid of flagella.

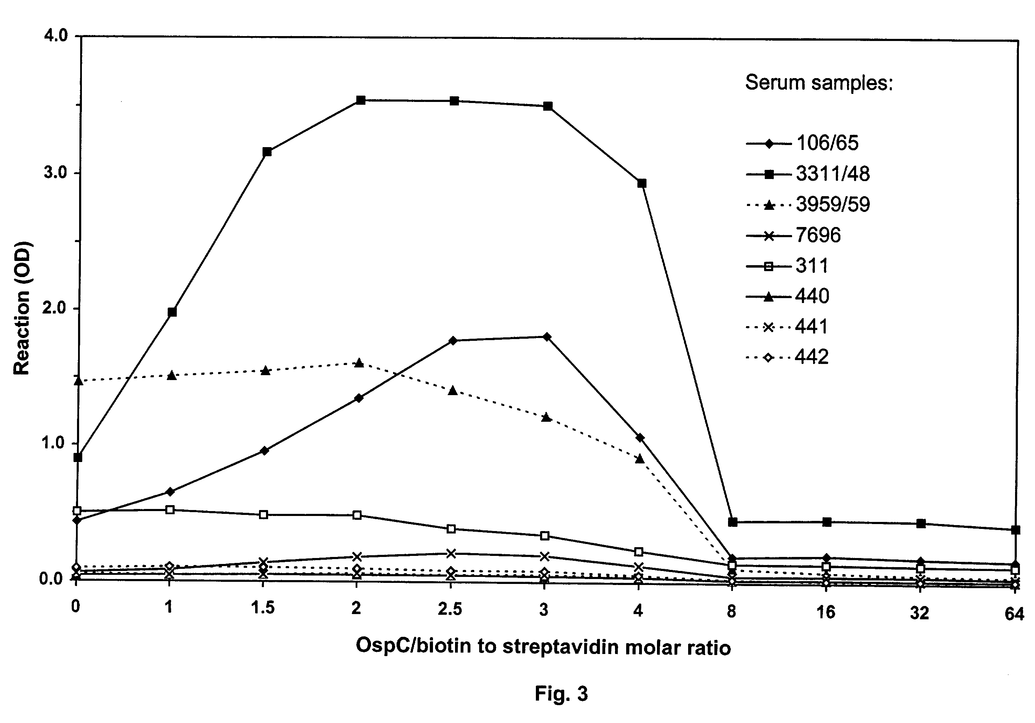

In the experiment just described, a synthetic OspC peptide representing the 23 most C-terminal amino acids was used. Results illustrated in Fig. 3 show that a peptide including only the 8 most C-terminal amino acids (see Example 1, Section A, peptide seq. no. 1) reacted in a way closely similar to the 23-mer peptide.

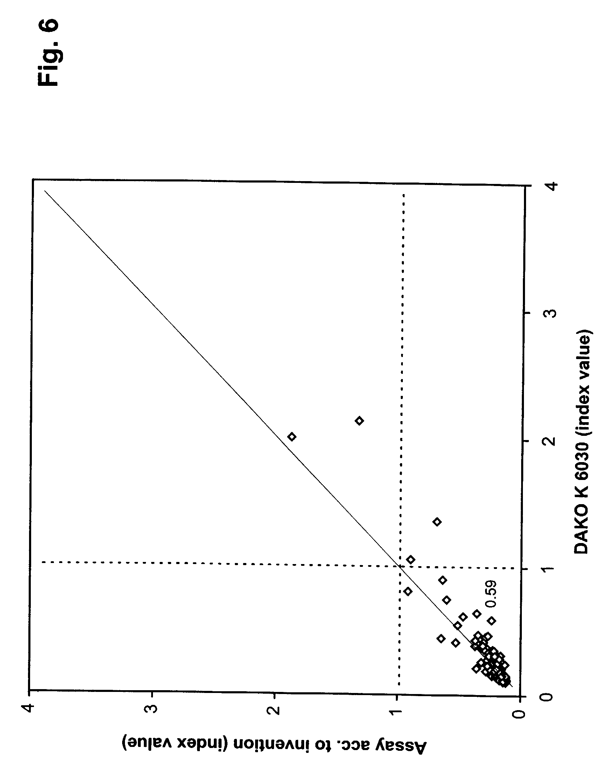

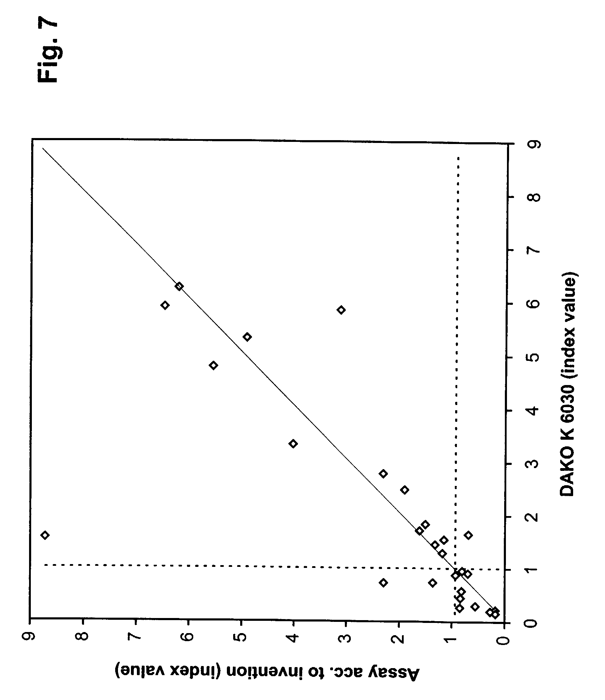

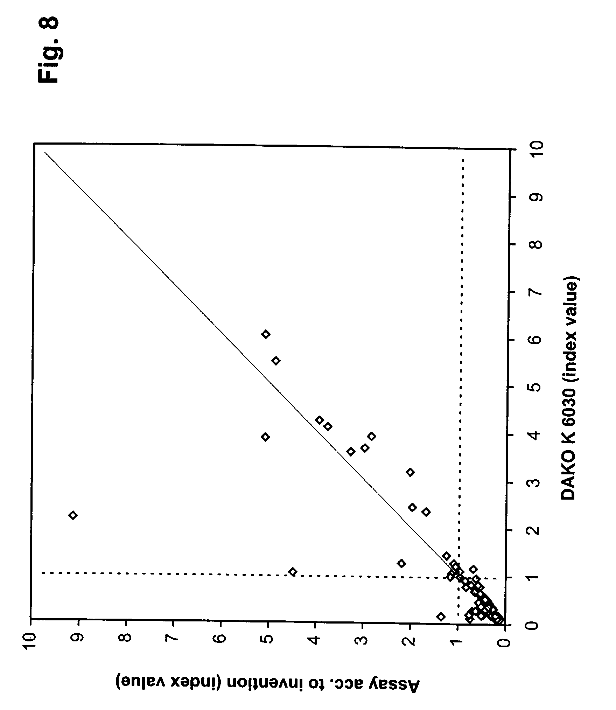

The diagnostic performance of the ELISA containing the above mentioned antigen complex of biotinylated flagellum, biotinylated OspC peptide, and streptavidin/HRP was further investigated. In this experiment, a stochiometric ratio of 3 of biotinylated OspC peptide (seq. no. 1) to streptavidin/HRP was applied because of its optimal (see Fig. 3) ability to bind anti-flagellum as well as anti-OspC IgM. Results from testing the panels of blood donor, *erythema migrans* and neuroborreliosis patient sera in this assay were compared to those obtained from testing the same panels in the IDEIA™ *Borrelia burgdorferi,* IgM, DAKO cat. no. K 6030. The results are illustrated in Figs. 6, 7 and 8. For each test sample an average of the OD value of duplicate wells was compared to the OD value of the assay cut-off control. The test sample OD was divided by the cut-off control OD, yielding an index value. An index value > 1 means positive, and ≤ negative. Fig. 6 shows that the assay according to the invention tests two out of 103 blood donor sera positive, while the K 6030 assay tests four out of 103 positive. Figs. 7 and 8 show that a few sera, negative or weakly positive in the K 6030 assay, are distinctly positive in the assay according to the invention, pointing to detection of anti-Ospc IgM in these samples.

Another antigen complex according to the invention comprises OspC peptides coupled to biotinylated latex particles (see Example 2, Section A), the particles being complexed with either streptavidin/HRP alone, or with streptavidin/HRP and flagellum/biotin, prior to their application in the µ-capture ELISA. Again the IDEIA™ *Borrelia burgdorferi,* IgM, cat. no. K 6030 was used, the only change being that the flagellum/biotin was replaced by latex particles to which is attached OspC peptides and biotin or both flagellum/biotin and latex particles to which is attached OspC peptides and biotin were used. Again the streptavidin/HRP final concentration was kept throughout the experiment such as applied in the original assay.

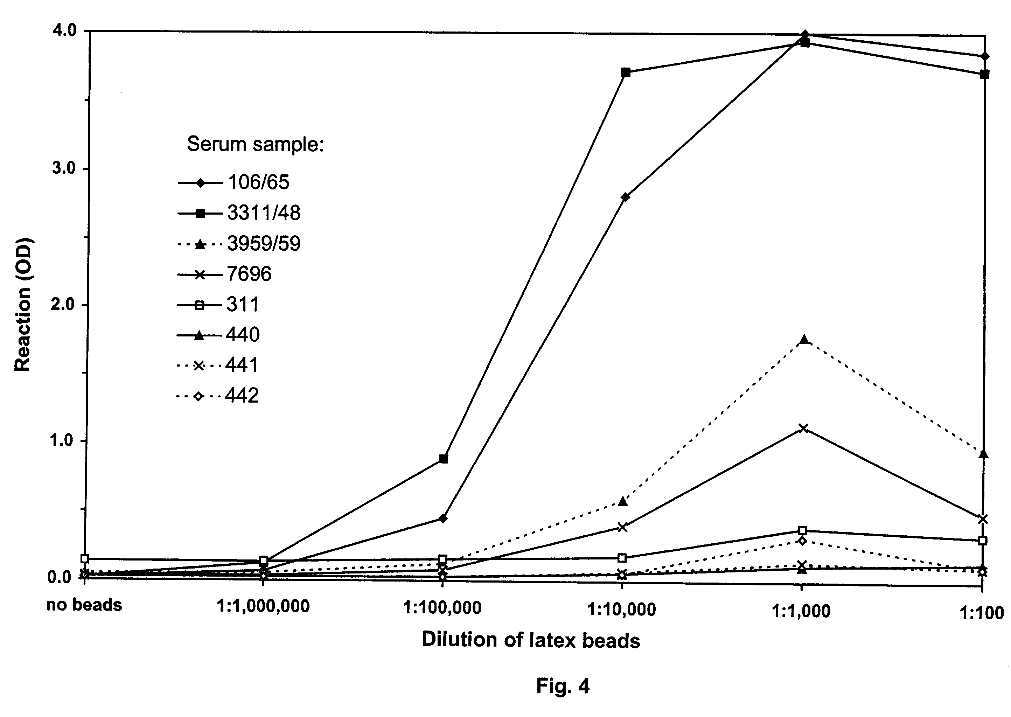

Fig. 4 shows the results obtained with a complex containing latex particles to which is attached OspC peptide seq. no. 3 and streptavidin/HRP, the density of particles being varied through a 10-fold dilution row ranging from a 10² to a 10⁶ dilution of the particle stock suspension. Included was also a control data point where no latex particles were applied, i.e. un-complexed streptavidin/HRP only was added to the wells. It will be seen that positive reactions (Fig. 4, curves for sera 106/65, 3311/48, 3959/59, 7696) clearly distinct from negative (Fig. 4, curves for sera 311, 440, 441, 442) were obtainable. Thus, four sera were positive for anti-OspC IgM and included those reacting positively for anti-OspC in the Western immunoblot assay (Fig. 1, column A) as well as in the µ-capture ELISA where an OspC peptide/biotin - flagellum/biotin - streptavidin/HRP complex was applied (Figs. 2 and 3).

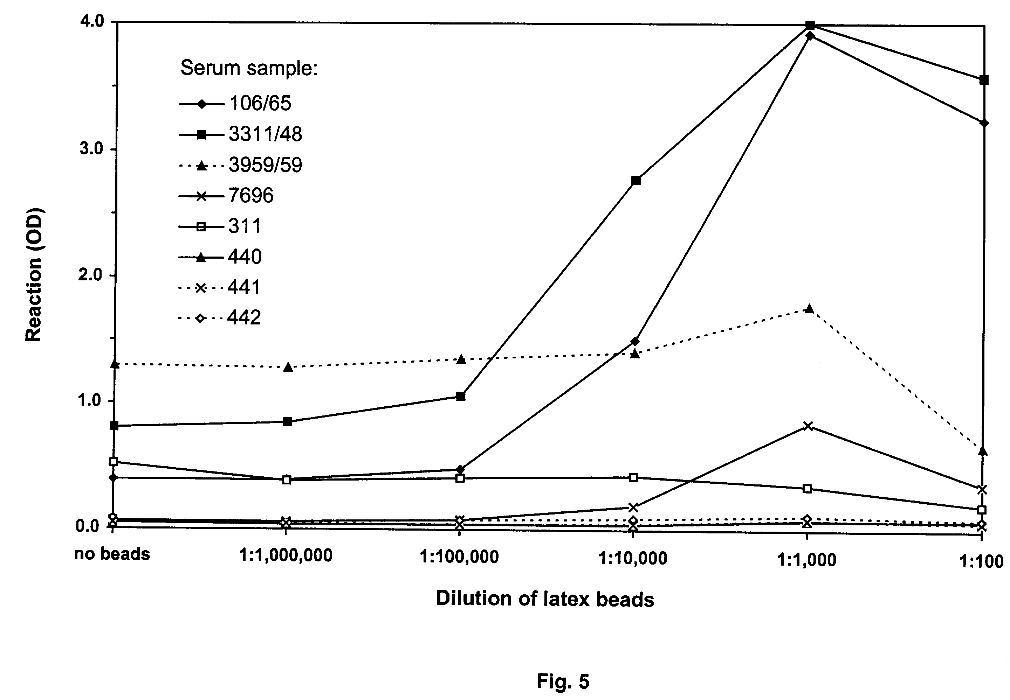

Fig. 5 shows the results obtained with a complex containing latex particles to which is attached OspC peptide seq. no. 3 and biotin, flagellum/biotin and streptavidin/HRP, the density of particles again being varied through a 10-fold dilution row ranging from a 10² to a 10⁶ dilution of the particle stock suspension. Included was also a control data point where no latex particles were applied, meaning that a complex consisting of flagellum/biotin and streptavidin/HRP only was added to the wells, resulting in an assay which in fact was the original µ-capture ELISA. This is clearly illustrated in Fig. 5, where the reactions obtained with the complex devoid of latex particles closely mimics those obtained with the IDEIA™ *Borrelia burgdorferi,* IgM, cat. no. K 6030 (Fig. 1, column D). Obvious is also that a complex including OspC peptide coupled onto biotinylated latex particles can identify anti-OspC as well as anti-flagellum IgM positive serum samples, given that the latex particle density is adjusted properly. In the present assay, a dilution of the stock suspension between 10³ and 10⁴ yielded the optimal results.

The serum sample 7696 gave in tests using antigen complexes comprising latex particles a higher signal as compare to what was found in the ELISA with an antigen complex containing biotinylated OspC and flagellum complexed with streptavidin/HRP (compare Figs. 4 and 5 with Figs. 2 and 3). This may indicate that a complex including latex particles with OspC is superior for detection of anti OspC.

### REFERENCES

1. Hansen et al., J. Clin. Microbiol., (1988), **26**, 338-46
2. Hansen et al., J. Clin. Microbiol., (1991), **29**, 166-173)
3. WO 97/42221 (Statens Serum Institut)
4. Gausepohl et al., Twenty-first European Peptide Symposium, Costa Brava, Spain, September 2-8, 1990)

## Claims

1. A method for determination of IgM antibodies against *Borrelia burgdorferi* (*B.* burgdorferi) sensu *lato* in a sample of human or animal fluid which method comprises the steps of
(i) contacting any sample antibodies to be detected with anti-IgM immobilised to a solid support,
(ii) optionally separating the solid support from the liquid phase,
(iii) contacting the antibodies bound to the solid support with an antigen complex comprising a carrrier to which is attached one or more sets of *Borrelia* OspC peptides and optionally one or more sets of other *Borrelia* peptides, each set comprising a plurality of peptides representing the same antibody binding epitope and binding to IgM molecules, which antigen complex is or will be labelled, and
(iv) determining the presence, if any, of antibodies against *B. burgdorferi sensu lato.*

2. A method for the determination of IgM antibodies against *B. burgdorferi sensu lato* in a sample of human or animal fluid which method comprises the steps of
(i) contacting any sample antibodies to be detected with anti-IgM immobilised to a solid support,
(ii) optionally separating the solid support from the liquid phase,
(iii) contacting the antibodies bound to the solid support with an antigen complex comprising a carrier to which is attached one or more sets of *Borrelia* OspC peptides, and an antigen complex comprising a carrier to which is attached one or more sets of other *Borrelia* peptides, each set comprising a plurality of *Borrelia* peptides representing the same antibody binding epitope and binding to IgM molecules, which antigen complex is or will be labelled, and
(iv) determining the presence, if any, of antibodies against *B. burgdorferi sensu lato.*

3. A method according to claim 1 or 2, wherein non-bound antigen complexes in step (iii) is removed before step (iv).

4. A method according to any one of claims 1-3, wherein the antibodies to be detected are in step (iii) are brought into contact with a labelled antigen complex comprising a carrier to which is attached one or more sets of *Borrelia* OspC peptides, each set being represented by a plurality of identical peptides.

5. A method according to any one of claims 1-4, wherein the carrier is a particle such as a latex particle, or a microsphere such as a metal particle, or a whole cell such as an erythrocyte.

6. A method according to any one of claims 1-4, wherein the carrier is an insoluble or soluble organic or inorganic polymer, the *Borrelia* flagellum or a fragment thereof.

7. A method according to any one of claims 1-4, wherein the carrier is a combination of a carrier according to claim 5 and a carrier according to claim 6.

8. A method according to any of claims 1 to 7, wherein the antigen complex is labelled by attaching a detectable moiety to the carrier.

9. A method according to claim 8, wherein the detectable moiety is covalently attached to the carrier.

10. A method according to any of claims 1 to 8, wherein the detectable moiety is attached to streptavidin which is bound to the carrier through biotin.

11. A method according to any of claims 1 to 10, wherein the detectable moiety is selected from the group consisting of chromophore, fluorophore, spin label, radioisotope, enzyme, hapten and chemiluminescent compounds.

12. A method according to any of claims 6 to 11, wherein the carrier is the *Borrelia* flagellum or fragments thereof.

13. A method according to any of claims 1 to 12, wherein the *Borrelia* peptides are bound covalently to the carrier.

14. A method according to any of claims 1 to 12, wherein the *Borrelia* peptides and the carrier are biotinylated and bound to each other through streptavidin.

15. A method according to any one of claims 1-14, wherein the carrier is the *Borrelia* flagellum or fragments thereof to which is attached a plurality of identical *Borrelia* OspC peptides, the flagellum or fragments thereof and the OspC peptides being biotinylated and attached to each other through streptavidin to which an enzyme is covalently attached.

16. A method according to any one of claims 1-14, wherein the carrier is a combination of a carrier according to claim 5 and the *Borrelia* flagellum or fragments thereof, the carrier components being biotinylated and attached to each other through streptavidin to which an enzyme is covalently attached, to which carrier according to claim 5 is attached a plurality of identical *Borrelia* OspC peptides.

17. A method according to any one of claims 1-16, wherein the antigen complex comprises *Borrelia* OspC peptides of a length of 5 to 25 amino acids and includes the C terminal end of OspC, and the enzyme attached to streptavidin is horseradish peroxidase.

18. A method according to any one of claims 1-3, wherein the antigen complex comprises a carrier to which is attached one or more sets of *Borrelia* peptides, each set being represented by a plurality of identical peptides.

19. A method according to any one of claims 1-18, wherein the length of the peptides are at most 60 amino acids, preferably at most 30 amino acids.

20. A method according to any one of claims 1-19, wherein the length of the peptides are from 2-30 amino acids, preferably from 4-30 amino acids, most preferably from preferably from 4-25 amino acids.

21. A method according to any one of claims 1-20, wherein amino acid sequence shares at least 60% homology with the native sequence.

22. A method according to any one of claims 1-21, wherein the length of the amino acid sequence is less than 10 amino acids, preferably from 2-9 amino acids, more preferably from 4-9 amino acids, most preferably 7 or 8 amino acids.

23. A method according to claim 22, wherein the amino acid sequence shares at least 80% homology with the native sequence.

24. A method according to any one of claims 1-23, wherein the *Borrelia* OspC peptides include the 4 C-terminal amino acids of OspC and have a length of less than 10 amino acids, preferably from 4-9 amino acids, most preferably 7 or 8 amino acids.

25. A method according to claim 24, wherein sequence of the *Borrelia* OspC peptides is
Val Ala Glu Ser Pro Lys Lys Pro (Seq. No. 1).

26. An antigen complex as defined in any of claims 1 to 25.

27. A kit for assaying antibodies against *B. burgdorferi sensu lato,* which kit comprises an antigen complex as defined in any of claims 1 to 25, and means enabling detection and/or measuring specific binding between the antigen complex and antibodies against *Borrelia* strains.

28. A kit according to claim 27, wherein the carrier of the antigen complex is the *Borrelia* flagellum or fragments thereof to which is attached a plurality of identical *Borrelia* OspC peptides, the flagellum or fragments thereof and the OspC peptides being biotinylated and attached to each other through streptavidin to which an enzyme is covalently attached.

29. A kit according to claim 27, wherein the carrier is a combination of a carrier according to claim 4 and the *Borrelia* flagellum or fragments thereof, the carrier components being biotinylated and attached to each other through streptavidin to which an enzyme is covalently attached, to which carrier according to claim 4 is attached a plurality of identical OspC peptides.
